(19) Europäisches Patentamt
European Patent Office
Office européen des brevets

(11) Veröffentlichungsnummer: **0 454 623 B1**

(12) **EUROPÄISCHE PATENTSCHRIFT**

(45) Veröffentlichungstag der Patentschrift: **13.09.95**

(51) Int. Cl.[6]: **C07C 45/72**, C07C 45/45, C07C 49/76

(21) Anmeldenummer: **91810289.8**

(22) Anmeldetag: **17.04.91**

Die Akte enthält technische Angaben, die nach dem Eingang der Anmeldung eingereicht wurden und die nicht in dieser Patentschrift enthalten sind.

# (54) Verfahren zur Herstellung von linearen 1,3-Diketonen.

(30) Priorität: **26.04.90 CH 1424/90**

(43) Veröffentlichungstag der Anmeldung:
**30.10.91 Patentblatt 91/44**

(45) Bekanntmachung des Hinweises auf die Patenterteilung:
**13.09.95 Patentblatt 95/37**

(84) Benannte Vertragsstaaten:
**BE DE ES FR GB IT NL**

(56) Entgegenhaltungen:
**DE-A- 1 618 442**
**DE-A- 1 618 444**
**US-A- 3 004 932**
**US-A- 4 482 745**

(73) Patentinhaber: **CIBA-GEIGY AG**
**Klybeckstrasse 141**
**CH-4002 Basel (CH)**

(72) Erfinder: **Drewes, Rolf, Dr.**
**Am Buchacker 1**
**W-6145 Lindenfels (DE)**
Erfinder: **Friedrich, Hans-Helmut**
**Am Rauhenstein 8**
**W-6147 Lautertal 2 (DE)**
Erfinder: **Mehner, Hans-Ludwig**
**Guldenweg 9**
**W-6840 Lampertheim (DE)**
Erfinder: **Braun, Bernd, Dr.**
**Im Heidenfeld 40**
**W-6147 Lautertal 3 (DE)**
Erfinder: **Wecht, Walter**
**Im Wiesental 4**
**W-6149 Rimbach (DE)**

**EP 0 454 623 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von linearen 1,3-Diketonen durch Claisen-Kondensation von Ketonen mit Estern in Gegenwart von Alkali- oder Erdalkalimetallhydriden oder -alkoholaten in einem Gemisch von Dimethylsulfoxid mit mindestens einem unter den Reaktionsbedingungen inerten organischen Lösungsmittel.

1,3-Diketone sind als wertvolle Costabilisatoren für chlorhaltige Polymerisate, insbesondere Polyvinylchlorid, die gegen schädigenden Einfluss von Warme und/oder Licht geschützt werden müssen, aus der Literatur bekannt. Ausserdem sind 1,3-Diketone wichtige Ausgangsstoffe und Zwischenprodukte zur Synthese von Heterocyclen. Die Claisen-Kondensation ist als Herstellungsmethode für 1,3-Diketone allgemein bekannt und in zahlreichen Lehrbüchern der organischen Chemie, wie z.B. Organikum, VEB Deutscher Verlag der Wissenschaften, Berlin (1969), S. 580, 632, 658, J.B. Hendrickson, D.J. Cram, G.S. Hammond, Organic Chemistry, McGraw-Hill S. 522, 524, 525 oder J. March, Advanced Organic Chemistry, J. Wiley & Sons (1985), S. 437-39, 835 beschrieben.

Normalerweise wird die Reaktion zur Herstellung der Diketone in einem inerten organischen Lösungsmittel in Gegenwart von Alkalialkoholaten als Base durchgeführt. So ist in der US-A 3,004,932 die Herstellung von ungesättigten β-Diketonen mit Alkalimetallalkoholaten in Diethylether veröffentlicht. Andere Veröffentlichungen beschreiben die Verwendung von Alkalimetallhydriden als Base bei der Claisen-Kondensation. Z.B. wird in J.P. Anselme, Org. Synth. Vol 32 (1967), 3716 Dibenzoylmethan via Claisen-Kondensation mit Natriumhydrid als Base in Dimethylsulfoxid hergestellt.

Cyclische β-Diketone werden in der DE-A 1 618 442 durch Claisen-Kondensation mit Alkalimetallalkoholaten in einer mindestens äquimolaren Menge Dimethylsulfoxid und evtl. einem weiteren inerten organischen Lösungsmittel hergestellt.

Da in den bekannten Verfahren der Claisen-Kondensation zum Erreichen guter Ausbeuten hohe Ueberschüsse an Keton eingesetzt werden müssen und lange Reaktionszeiten erforderlich sind, besteht ein Interesse an verbesserten Verfahren zur Durchführung dieser Reaktion. Ausserdem ist die bisher angewandte übliche Methode der Isolierung und Reinigung der synthetisierten Diketone aufwendig. So ist es für die industrielle Anwendung von Vorteil, ein vereinfachtes Verfahren einzuführen.

Es wurde nun überraschenderweise gefunden, dass durch die Verwendung eines Gemisches von Dimethylsulfoxid mit einem weiteren, unter den Reaktionsbedingungen inerten, organischen Lösungsmittel das Verfahren besonders vorteilhaft abläuft. Es wird die Reaktionszeit verkürzt und eine sehr hohe Ausbeute erzielt.

Gegenstand der Erfindung ist ein Verfahren zur Herstellung von linearen 1,3-Diketonen der allgmeinen Formel I

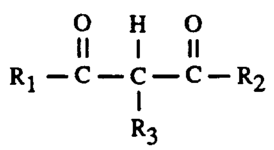

(I)

worin

$R_1$ und $R_2$ unabhängig voneinander für $C_1$-$C_{20}$-Alkyl, Phenyl, durch Halogen, Hydroxy, $NO_2$, $C_1$-$C_4$-Alkyl und/oder $C_1$-$C_4$-Alkoxy substituiertes Phenyl, $C_7$-$C_9$-Phenylalkyl oder für einen Rest der Formel II stehen

$-A-X-R_4$ (II)

wobei

A $C_1$-$C_{12}$-Alkylen, Phenylen, durch Halogen, Hydroxy, $NO_2$, $C_1$-$C_4$-Alkyl und/oder $C_1$-$C_4$-Alkoxy substituiertes Phenylen oder durch Hydroxy, Halogen oder/und Alkoxy substituiertes $C_1$-$C_{12}$-Alkylen bedeutet,

X für Sauerstoff oder Schwefel steht und

$R_4$ Wasserstoff, $C_1$-$C_{18}$-Alkyl, Phenyl, durch Halogen, Hydroxy, $C_1$-$C_4$-Alkyl, $NO_2$ und/oder $C_1$-$C_4$-Alkoxy substituiertes Phenyl oder $C_7$-$C_9$-Phenylalkyl bedeutet und $R_3$ Wasserstoff, $C_1$-$C_{20}$-Alkyl, Phenyl, durch Halogen, Hydroxy, $C_1$-$C_4$-Alkyl, $NO_2$ und/oder $C_1$-$C_4$-Alkoxy substituiertes Phenyl oder $C_7$-$C_9$-Phenylalkyl darstellt, durch Claisen-Kondensation von Ketonen der Formel III

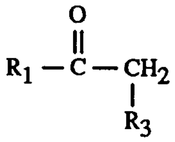

(III)

mit Estern der Formel IV

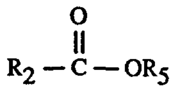

(IV)

worin $R_5$ für $C_1$-$C_5$-Alkyl, Phenyl, oder durch Halogen, $C_1$-$C_4$-Alkyl oder Hydroxy substituiertes Phenyl steht;
oder, wenn $R_2$ in Formel I $-(CH_2)_mOH$ bedeutet, auch mit cyclischen Estern der Formel V

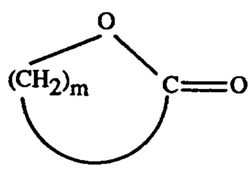

(V)

in der m 2 bis 10 bedeutet,
in Gegenwart eines Alkali- oder Erdalkalimetallhydrids oder $C_1$-$C_5$-Alkali- oder Erdalkalimetallakoholats als Base, dadurch gekennzeichnet, dass man die Umsetzung in einem Gemisch von Dimethylsulfoxid mit mindestens einem unter den Reaktionsbedingungen inerten, organischen Lösungmittel durchführt, wobei als inerte organische Lösungsmittel Dioxan, Tetrahydrofuran, Diethylenglycoldimethylether, Methyl-t-butylether, Toluol oder N-Methylpyrrolidon eingesetzt werden.

$R_1$ und $R_2$ als $C_1$-$C_{20}$-Alkyl können linear oder verzweigt sein und beispielsweise Methyl, Ethyl, n-Propyl, Isopropyl, n-Butyl, tert-Butyl, n-Pentyl, Isopentyl, n-Hexyl, n-Heptyl, n-Octyl, Isooctyl, n-Nonyl, Decyl, Undecyl, Dodecyl, Tridecyl, Tetradecyl, Pentadecyl, Hexadecyl, Heptadecyl, Octadecyl, Nonadecyl oder Eicosyl, bevorzugt $C_1$-$C_{18}$-Alkyl, insbesondere z.B. Methyl, Isopentyl, n-Nonyl, Pentadecyl oder Heptadecyl bedeuten.

Ist $R_1$ oder $R_2$ substituiertes Phenyl, enthält letzteres z.B. 1 bis 3, insbesondere 1 oder 2 Substituenten, vorzugsweise einen Substituenten.

$R_1$ und $R_2$ als ($C_1$-$C_4$-Alkyl)-phenyl können beispielsweise für mit 1-3, insbesondere 1 oder 2 Alkylgruppen, vor allem mit Methylgruppen substituiertes Phenyl stehen. Beispiele dafür sind Tolyl, Xylyl oder Mesityl.

$R_1$ und $R_2$ als durch Halogen substituiertes Phenyl können beispielsweise einen ein- oder mehrfach durch Fluor, Chlor oder Brom, insbesondere Chlor oder Brom substituierten Phenylring bedeuten, wie z.B. Chlorphenyl oder Dichlorphenyl.

$C_1$-$C_4$-Alkoxy bedeutet beispielsweise Methoxy, Ethoxy, Propyloxy oder Butoxy, ein entsprechend substituiertes Phenyl ist beispielsweise Methoxyphenyl.

$R_1$ und $R_2$ als $C_7$-$C_9$-Phenylalkyl stehen z.B. für Benzyl, Phenylethyl, $\alpha$-Methylbenzyl, 3-Phenylpropyl oder $\alpha,\alpha$-Dimethylbenzyl, bevorzugt ist Benzyl. Bevorzugt sind $R_1$ und $R_2$ $C_1$-$C_{18}$-Alkyl, Phenyl, ($C_1$-$C_4$-Alkyl)phenyl oder $-A-X-R_4$.

A als $C_1$-$C_{12}$-Alkylen kann beispielsweise ein lineares oder verzweigtes, vorzugsweise lineares Alkylen bedeuten. Beispiele für solche Reste ergeben sich aus den obigen Beispielen für Alkyl als $R_1$ und $R_2$ bis zur entsprechenden Anzahl der C-Atome durch Anfügen des Suffixes -en. Bevorzugt ist $C_1$-$C_6$-Alkylen, insbesondere n-Propylen oder n-Pentylen.

$R_4$ als $C_1$-$C_{18}$-Alkyl kann z.B. für lineares oder verzweigtes Alkyl stehen wie beispielsweise für $R_1$ und $R_2$ beschrieben bis zur entsprechenden Anzahl der C-Atome.

$R_4$ als substituiertes Phenyl oder $C_7$-$C_9$-Phenylalkyl kann die gleichen Bedeutungsmöglichkeiten haben wie für $R_1$ und $R_2$ beschrieben.

$R_4$ ist bevorzugt Wasserstoff, $C_1$-$C_{18}$-Alkyl oder Phenyl.

A als gegebenenfalls substituiertes Phenylen ist vorzugsweise o- oder p-Phenylen, insbesondere unsubstituiertes Phenylen.

$R_3$ als $C_1$-$C_{20}$-Alkyl, substituiertes Phenyl oder $C_7$-$C_9$-Phenylalkyl kann die gleichen Bedeutungsmöglichkeiten haben wie für $R_1$ und $R_2$ beschrieben, bevorzugt ist $C_1$-$C_4$-Alkyl.

$R_3$ steht bevorzugt für Wasserstoff oder $C_1$-$C_4$-Alkyl, ganz besonders bevorzugt ist $R_3$ Wasserstoff.

$R_5$ als $C_1$-$C_5$-Alkyl bedeutet beispielsweise Methyl, Ethyl, n-Propyl, Isopropyl, n-Butyl, tert-Butyl, n-Pentyl oder Isopentyl. $R_5$ ist insbesondere $C_1$-$C_4$-Alkyl, vor allem Methyl.

$R_5$ als ($C_1$-$C_4$-Alkyl)-phenyl kann die gleichen Bedeutungen haben wie für $R_1$ und $R_2$ beschrieben.

Bei dem verwendeten Alkalimetallhydrid handelt es sich beispielsweise um Lithiumhydrid, Natriumhydrid oder Kaliumhydrid, insbesondere Natriumhydrid und Kaliumhydrid, ganz besonders bevorzugt um Natriumhydrid.

Beispiele für Erdalkalihydride sind Magnesiumhydrid und Calciumhydrid. Bevorzugt sind Alkalimetallhydride.

Beispiele für $C_1$-$C_5$-Alkalimetallalkoholate sind $LiOCH_3$, $NaOCH_3$, $KOCH_3$, $LiOC_2H_5$, $NaOC_2H_5$, $KOC_2H_5$, LiOn-$C_3H_7$ NaOn-$C_3H_7$, KOn-$C_3H_7$, LiOi-$C_3H_7$, NaOi-$C_3H_7$, KOi-$C_3H_7$, LiOn-$C_4H_9$, NaOn-$C_4H_9$, KOn-$C_4H_9$, LiOi-$C_4H_9$, NaOi-$C_4H_9$, KOi-$C_4H_9$, LiOtert-$C_4H_9$, NaOtert-$C_4H_9$, KOtert-$C_4H_9$, LiOn-$C_5H_{11}$, NaOn-$C_5H_{11}$, KOn-$C_5H_{11}$, LiOi-$C_5H_{11}$, NaOi-$C_5H_{11}$, KOi-$C_5H_{11}$, LiOtert-$C_5H_{11}$, NaOtert-$C_5H_{11}$, KOtert-$C_5H_{11}$.

Entsprechende Erdalkalimetallalkoholate sind beispielsweise $Mg(OCH_3)_2$, $Ca(OCH_3)_2$, $Mg(OC_2H_5)_2$, $Ca(OC_2H_5)_2$, Mg(On-$C_3H_7$)$_2$, Ca(On-$C_3H_7$)$_2$, Mg(Oi-$C_3H_7$)$_2$, Ca(Oi-$C_3H_7$)$_2$, Mg(On-$C_4H_9$)$_2$, Ca(On-$C_4H_9$)$_2$, Mg(Otert-$C_4H_9$)$_2$, Ca(Otert-$C_4H_9$)$_2$, Mg(Oi-$C_4H_9$)$_2$, Ca(Oi-$C_4H_9$)$_2$, Mg(On-$C_5H_{11}$)$_2$, Ca(On-$C_5H_{11}$)$_2$, Mg(Oi-$C_5H_{11}$)$_2$, Ca(Oi-$C_5H_{11}$)$_2$, Mg(Otert-$C_5H_{11}$)$_2$, Ca(Otert-$C_5H_{11}$)$_2$, insbesondere Magnesiumalkoholate.

Bevorzugt werden Alkalimetallalkoholate, insbesondere Na-Alkoholate, z.B. $NaOCH_3$, $NaOC_2H_5$ und NaOtert-$C_4H_9$, insbesondere $NaOCH_3$ und NaOtert-$C_4H_9$, verwendet.

Als inerte organische Lösungsmittel werden Dioxan, Tetrahydrofuran, Diethylenglycoldimethylether, Methyl-tert-butylether, Toluol oder N-Methylpyrrolidon verwendet.

Von besonderem Interesse ist die Herstellung von Verbindungen der Formel I, worin
$R_1$ und $R_2$ unabhängig voneinander $C_1$-$C_{20}$-Alkyl, Phenyl, ($C_1$-$C_4$-Alkyl)-phenyl oder einen Rest der Formel II bedeuten,
A für $C_1$-$C_6$-Alkylen steht,
$R_4$ Wasserstoff, $C_1$-$C_{18}$-Alkyl, Phenyl oder ($C_1$-$C_4$-Alkyl)-phenyl darstellt und
$R_3$ für Wasserstoff und $C_1$-$C_4$-Alkyl steht.

Bevorzugt werden Verbindungen der Formel I beansprucht, worin
$R_1$ und $R_2$ unabhängig voneinander $C_1$-$C_{18}$-Alkyl, Phenyl oder einen Rest der Formel II bedeuten,
$R_4$ Wasserstoff, Phenyl oder $C_1$-$C_{18}$-Alkyl darstellt und
$R_3$ für Wasserstoff steht.

Erfindungswesentlich ist, dass im oben beschriebenen Verfahren ein Lösungsmittelgemisch aus Dimethylsulfoxid und mindestens einem der oben genannten unter den Reaktionsbedingungen inerten organischen Lösungsmittel verwendet wird. Bevorzugt sind Gemische, in denen der Anteil von Dimethylsulfoxid z.B. 10-80 %, vorzugsweise 10-60 %, insbesondere 15-50 %, beträgt. Auch Gemische mit 20-70 %, z.B. 30-60 % Dimethylsulfoxid sind zweckmässig.

Bevorzugt sind Lösungsmittelgemische, die kein Dimethylformamid enthalten. Das Reaktionsgemisch kann auch geringe Anteile des zum jeweiligen Alkali- oder Erdalkalialkoholat kortespondierenden Alkohols enthalten.

Zweckmässig ist die Durchführung des erfindungsgemässen Verfahrens bei Temperaturen zwischen -20 und +70 °C. Bevorzugt liegen die Reaktionstemperaturen z.B. zwischen -5 und +40 °C.

Die Reaktionszeiten des oben beschriebenen Verfahrens zur Claisen-Kondensation können in weiten Grenzen schwanken, liegen im allgemeinen aber zwischen 0,5 und 5,0 Stunden.

Von Bedeutung ist auch die Möglichkeit einer vereinfachten Aufarbeitung nach der Reaktion. Sie besteht darin, dass das aus der Reaktionslösung ausfallende Alkali- oder Erdalkalimetallsalz des Diketons direkt aus der Lösung abfiltriert und gewaschen wird, und danach durch Hydrolyse mit einer verdünnten Säure das reine Diketon erhalten wird. Als Säuren kommen beispielsweise Essigsäure, Ameisensäure, Phosphorsäure, Salzsäure oder Schwefelsäure in Frage, bevorzugt sind Salzsäure und Schwefelsäure.

Wie bereits eingangs erwähnt, stellen die erfindungsgemäss herstellbaren linearen 1,3-Diketone wertvolle Costabilisatoren für chlorhaltige Polymerisate, die gegen schädigenden Einfluss von Wärme und/oder Licht geschützt werden müssen, dar. Es besteht daher ein Interesse daran diese Diketone in möglichst

einfachen Verfahren mit wenig Energieaufwand in hohen Ausbeuten herzustellen.

Das erfindungsgemässe Verfahren eröffnet einen technisch besonders günstigen und wirtschaftlichen Weg zu deren Herstellung.

Ein wichtiger Vorteil des erfindungsgemässen Verfahrens liegt darin, dass zu seiner Durchführung relativ niedrige Reaktionstemperaturen erforderlich sind, ein wichtiger Aspekt der Energieersparnis für die industrielle Anwendung. So kann das Verfahren z.B. bei -20-70 °C vorzugsweise bei -5° bis +40 °C durchgeführt werden. Eine Erhöhung der Reaktionstemperaturen hat nicht zwangsweise eine Ausbeutesteigerung zur Folge. In den bisher bekannten Verfahren ist die Verwendung eines bis zu 100%igen Ueberschusses an Keton zur Erlangung guter Ausbeuten erforderlich. Als besonderer technischer Vorteil des erfindungsgemässen Verfahrens ist daher auch die mögliche Erniedrigung des Edukt- und Basen-Ueberschusses im Vergleich zu den bisher bekannten Verfahren zu nennen, was eine Verringerung der Entsorgungsprobleme mit sich bringt. So ist es möglich, mit dem erfindungsgemässen Verfahren auch mit annähernd stöchiometrischen Mengen oder geringen Ueberschüssen an Ester hohe Ausbeuten zu erzielen. Die Esterkomponente und/oder die Base setzt man zweckmässig in einer Menge von 0,5-1,5 Mol, vorzugsweise 0,65-1,25 Mol, insbesondere 0,9-1,2 Mol, bezogen auf 1 Mol Keton, ein.

Die Reaktion wird vorteilhaft so durchgeführt, dass man z.B. die Base im Lösungsmittel vorlegt und die Ester- und Ketonkomponente nacheinander oder gleichzeitig zugibt. Uebliche Operationen wie Rühren des Reaktionsgemisches sind von Vorteil.

Das erfindungsgemässe Verfahren beinhaltet in einer bevorzugten Ausführungsform auch eine vereinfachte Möglichkeit der Aufarbeitung des Reaktionsproduktes. So braucht dieses nicht unbedingt, wie bisher bekannt, noch in der Reaktionslösung hydrolysiert und dann mit Hilfe von organischen Lösungsmitteln extrahiert zu werden, sondern das Alkali- oder Erdalkalimetallsalz des Produktes fällt aus der Reaktionslösung aus (gegebenenfalls nach Abdestillieren zumindest eines Teiles des Lösungsmittels und gegebenenfalls nach Zugabe eines Lösungsmittels, in dem das Salz nicht löslich ist), wird abfiltriert, gewaschen und dann das reine isolierte Salz hydrolisiert. Ein Vorteil liegt darin, dass das organische Lösungsmittel nicht mit Wasser verunreinigt wird und leicht wiederverwendet werden kann.

Ausserdem werden durch diese Aufarbeitungsweise Produkte mit hoher Reinheit erhalten, da entstandene organische Nebenprodukte zusammen mit dem organischen Lösungsmittel abgetrennt werden können, während diese Produkte bei der Aufarbeitung mittels Extraktion mit dem gewünschten Reaktionsprodukt zusammen extrahiert werden und dieses verunreinigen.

Wie dem Fachmann bekannt, können die Verbindungen der Formel I selbstverständlich teilweise oder ganz in den tautomeren Formen vorliegen nach dem Gleichgewicht

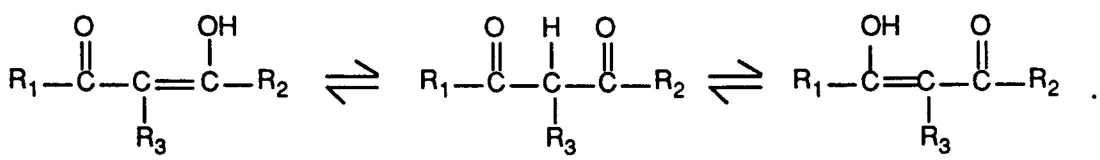

Die nachfolgenden Beispiele erläutern das erfindungsgemässe Verfahren weiter. Darin, sowie in der übrigen Beschreibung und den Patentansprüchen bedeuten Teile Gewichtsteile und Prozentangaben Gewichtsprozent, sofern nichts anderes angegeben ist.

Beispiel 1: 1,3-Diphenyl-propan-1,3-dion (Dibenzoylmethan)

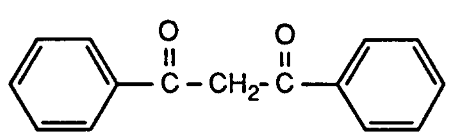

In einem 0,5 l Sovirel-Kolben (doppelwandiges Reaktionsgefäss) mit Rührer, Thermometer, Tropftrichter und Rückflusskühler mit Blasenzähler werden unter Stickstoff je 100 g wasserfreies Dimethylsulfoxid (DMSO) und Tetrahydrofuran und 10 g Natriumhydrid (80 % in Paraffinöl) auf ~5 °C abgekühlt und ein Gemisch aus 45 g Benzoesäuremethylester/36 g Acetophenon innerhalb 60 Minuten bei 5-10 °C eingetropft. Nach beendeter Zugabe wird das Gemisch noch 10 Minuten bei 5 °C nachgerührt, anschliessend auf 30 °C erwärmt, in einen 1-Liter-Einhalskolben übergespült und am Rotationsverdampfer die niedrigsiedenden Anteile abdestilliert. Der Rückstand von 203 g wird in 800 ml Eiswasser gelöst, mit 30 g 50%iger Schwefelsäure angesäuert, 10 Minuten gerührt, die ausgefallenen Kristalle abgesaugt, mit Wasser gewaschen und bis zur Gewichtskonstanz getrocknet.

Ausbeute: 63,6 g ≙ 94,5 % der Theorie, schwach gelbe Kristalle mit einem Schmelzpunkt von 72-75 °C.
Gehalt laut Gaschromatogramm 98,2 % Dibenzoylmethan.

Beispiel 2: 1,3-Diphenyl-propan-1,3-dion (Dibenzoylmethan)

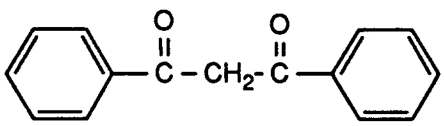

In einer Apparatur wie in Beispiel 1 beschrieben werden 100 g wasserfreies Dimethylsulfoxid, 100 g Diethylenglycoldimethylether, 6 g absolutes Methanol und 18 g Natriummethylat (97%ig) vorgelegt. Zu dieser Mischung wird bei 25 °C ein Gemisch aus 45 g Benzoesäuremethylester und 36 g Acetophenon innerhalb von 45 min. zugetropft. Nach der Zudosierung wird das Reaktionsgemisch 2 h bei 30 °C nachgerührt und anschliessend das Lösungsmittel unter Vakuum bei einer Temperatur von < 60 °C abdestilliert. Der Rückstand wird mit 150 g Methyl-tert-butylether verdünnt, auf 0 °C abgekühlt und filtriert. Der Filterkuchen wird zweimal mit Methyl-tert-butylether gewaschen. Anschliessend wird der leicht grau gefärbte Feststoff in verdünnter Salzsäure aufgenommen, gerührt und nach dem Abfiltrieren bis zur Gewichtskonstanz getrocknet.

Ausbeute: 61,3 g ≙ 91,1 % der Theorie weisse bis leicht gelbe Kristalle mit einem Schmelzpunkt von 72-75 °C.

Beispiel 3: 1-Phenyl-butan-1,3-dion

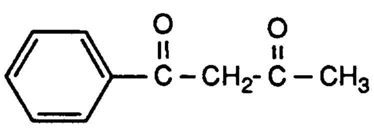

Es wird wie in Beispiel 1 verfahren, wobei an Stelle von Acetophenon 17,4 g Aceton eingesetzt werden und die Lösungsmittelmenge von 100 g auf 150 g Dimethylsulfoxid/Tetrahydrofuran erhöht wird.

Ausbeute: 42,6 g ≙ 87,5 % der Theorie, gelbes kristallines Pulver mit einem Schmelzpunkt von 52-53 °C.

Laut Kernresonanzspektrum liegt ein Gemisch von 14 %

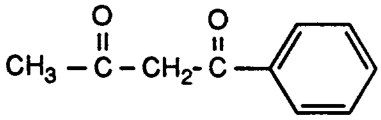

und 86 %

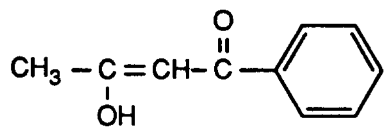

vor.

Beispiel 4: 6-Methyl-1-phenyl-heptan-1,3-dion

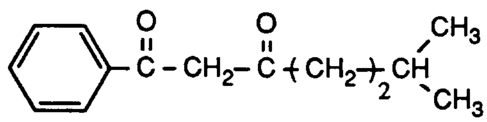

Verfahren wie im Beispiel 1 beschrieben; anstatt Acetophenon werden 34,5 g 5-Methyl-2-hexanon eingesetzt.

Die Aufarbeitung nach dem Ansäuern des Reaktionsgemisches mit Schwefelsäure erfolgt durch Extrahieren der wässrigen Lösung mit 2 x 100 ml Dichlormethan, anschliessende Trocknung über Natriumsulfat, Abziehen des Lösungsmittels am Rotationsverdampfer und Reinigung durch Destillation.

Ausbeute: 52,8 g ≙ 80,6 % der Theorie einer gelben Flüssigkeit mit einem Siedepunkt 98-100 °C/0,2 mbar und einem Brechungsindex $n_D^{20}$ : 1,5505.

Beispiel 5: 1-Phenyl-dodecan-1,3-dion

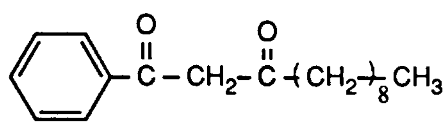

Verfahren wie im Beispiel 1 beschrieben; anstatt Acetophenon werden 51,0 g 2-Undecanon eingesetzt.

Die Aufarbeitung erfolgt wie im Beispiel 4 beschrieben.

Ausbeute: 74,6 g ≙ 90,7 % der Theorie einer farblosen Flüssigkeit mit einem Siedepunkt 140-42 °C/0,25 mbar und einem Schmelzpunkt von 36-37 °C.

Beispiel 6: 1-Phenyl-6-n-dodecylthio-hexan-1,3-dion

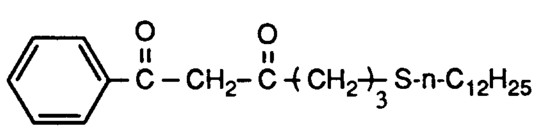

Verfahren wie im Beispiel 1 beschrieben; es werden jedoch 24 g Acetophenon eingesetzt und anstatt Benzoesäuremethylester und Natriumhydrid werden 66,6 g n-Dodecylthiobuttersäuremethylester und 21,2 g Natrium-t-butylat eingesetzt. Das Tetrahydrofuran wird durch die gleiche Menge Toluol ersetzt und die Reaktionstemperatur beträgt 0 °C.

Ausbeute nach Umkristallisation aus i-Propanol/Wasser: 55,7 g ≙ 71,4 % der Theorie, weisse Kristalle mit einem Schmelzpunkt von 44 °C.

Beispiel 7: 1-Phenyl-6-phenylthio-hexan-1,3-dion

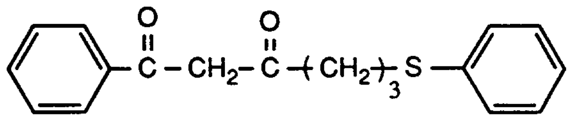

Verfahren wie im Beispiel 1 beschrieben; es werden 18 g Acetophenon und 4,9 g Natriumhydrid eingesetzt und der Benzoesäuremethylester wird durch 34,7 g Phenylthiobuttersäuremethylester ersetzt. Die Aufarbeitung erfolgt wie im Beispiel 4 beschrieben.

Ausbeute: 33,4 g ≙ 74,4 % der Theorie einer gelben Flüssigkeit mit einem Siedepunkt 178-82 °C/0,13 mbar und einem Brechungsindex $n_D^{20}$ : 1,6185.

Beispiel 8: 8-Hydroxy-1-phenyl-octan-1,3-dion

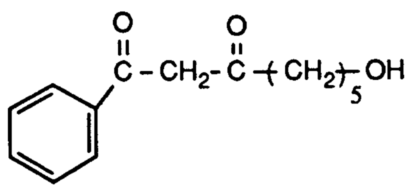

In einem 0,5-l Sovirel-Kolben (doppelwandiges Reaktionsgefäss) mit Rührer, Thermometer, Tropftrichter und Destillationsvorlage werden 99,0 g Natriummethylatlösung (30%ige Lösung in Methanol) und 117 g Dimethylsulfoxid unter Stickstoff vorgelegt. Aus dieser Lösung werden ca. 62 g Methanol abdestilliert. Die entstandene Suspension wird abgekühlt. Bei 30 °C werden 100 g N-Methyl-pyrrolidon zugegeben und auf 0 °C abgekühlt. Bei dieser Temperatur wird eine Lösung von 60,0 g Acetophenon, 62,8 g ε-Caprolacton und 17,0 g N-Methyl-Pyrrolidon im Verlauf einer Stunde zudosiert. Dann wird 30 Minuten lang bei 0 °C, anschliessend 2 Stunden bei 20 °C nachgerührt. Aus der Produktlösung werden bei einer Sumpftemperatur von <70 °C ca. 120 g Lösungsmittelgemisch abdestilliert Nach Abkühlung auf 30 °C werden 300 ml Wasser zum Rückstand gegeben. Die Lösung wird dreimal mit je 70 ml Xylol extrahiert, mit Wasser auf 1200 ml verdünnt und dann mit ca. 28 ml 50%iger Schwefelsäure auf pH 5,5 eingestellt. Das ausgefallene Produkt wird abfiltriert, auf der Nutsche mit 100 ml Wasser gewaschen und im Vakuum bei ca. 30 °C bis zur Gewichtskonstanz getrocknet.

Ausbeute: 85,0 g ≙ 72,6 % der Theorie leicht gelbliche Kristalle mit einem Schmelzpunkt von 46-48 °C. Gehalt laut Gaschromatogramm: 97,3 %.

Beispiel 9: 6-Hydroxy-1-phenyl-hexan-1,3-dion

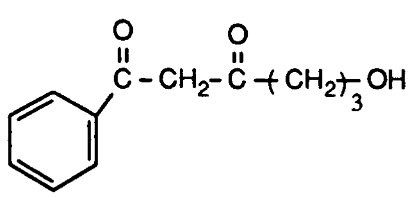

In gleicher Weise wie unter Beispiel 8 beschrieben, werden 60,0 g Acetophenon, 47,4 g Butyrolacton und 99,0 g Natriummethylatlösung (30%ige Lösung in Methanol) umgesetzt. Nach der unter Beispiel 8

beschriebenen Aufarbeitung fällt das Produkt nach mehreren Stunden aus der Mutterlauge aus, wird abfiltriert, mit Wasser gewaschen und im Vakuum bei ca. 30 °C bis zur Gewichtskonstanz getrocknet.

Ausbeute: 70,0 g ≙ 68 % der Theorie, gelbe Kristalle mit einem Schmelzpunkt von 35 °C.

Beispiel 10: 8-Hydroxy-1-phenyl-octan-1,3-dion

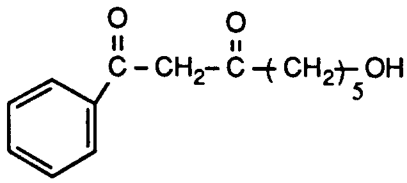

In einer Apparatur wie in Beispiel 8 werden 30,8 g Natriummethylat (97 %), 117,0 g DMSO und 117,0 g Dioxan vorgelegt und auf 0 °C gekühlt. Bei dieser Temperatur wird eine Lösung von 61,3 g Acetophenon, 57,7 g ε-Caprolacton und 17,0 g Dioxan unter Rühren im Verlauf einer Stunde zudosiert. Es wird 30 Minuten bei 0 °C und anschliessend 2 Stunden bei 20 °C nachgerührt. Dann werden bei 70 °C und vermindertem Druck ca. 220 g Lösungsmittel abdestilliert.

Der Rückstand wird mit 300 g Wasser versetzt und die Suspension dreimal mit je 70 ml Xylol extrahiert. Dann wird die wässrige Phase auf 1000 ml verdünnt, mit Schwefelsäure auf pH ~ 5 eingestellt. Das ausgefallene Produkt wird abfiltriert und getrocknet.

Ausbeute: 78,1 g ≙ 66,6 % der Theorie gelbliche Kristalle mit einem Schmelzpunkt von 47-50 °C.

Wenn das Natriummethylat durch 52,9 g Natrium-tert.-butylat ersetzt wird, beträgt die Ausbeute 79,0 g ≙ 67,4 % der Theorie.

Beispiel 11: 8-Hydroxy-1-phenyl-octan-1,3-dion

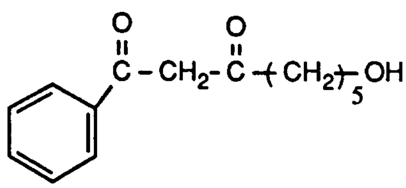

Analog zu Beispiel 8 werden in 117,0 g N-Methylpyrrolidon als Lösungsmittel 61,3 g Acetophenon und 57,7 g ε-Caprolacton mit 52,9 g Natrium-tert-butylat (97 %) als Base kondensiert Es wird wei bei Beispiel 8 beschrieben aufgearbeitet.

Ausbeute: 73,0 g ≙ 62,3 % der Theorie. Gelbliche Kristalle vom Schmelzpunkt 47-50 °C.

Beispiel 12: 1,3-Diphenyl-propan-1,3-dion

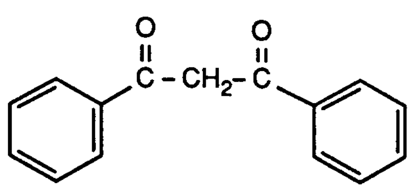

In einer Apparatur wie in Beispiel 1 werden 18,0 g Natriumethylat (97 %), 100 g DMSO, 100 g Dioxan und 6 g Methanol vorgelegt und auf 0 °C abgekühlt. Anschliessend werden 45 g Benzoesäuremethylester und 36 g Acetophenon als Gemisch über einen Zeitraum von 35 Minuten zugetropft. Das Reaktionsgemisch wird dann ca. 45-60 Minuten bei 30 °C gerührt.

Aufarbeitung Methode A:

Das Lösungsmittel wird am Rotationsverdampfer abdestilliert (Badtemperatur <60 °C) und der Rückstand mit 800 ml Wasser gelöst. Anschliessend wird mit verdünnter Salzsäure angesäuert und der Niederschlag filtriert, mit Wasser gewaschen und getrocknet.

Ausbeute: 60,0 g ≙ 89,2 % der Theorie.
Reinheit: >98 % nach $^{13}$C-Kernresonanzspektrum.
Schmelzpunkt: 72-75 °C.

Aufarbeitung Methode B:

Das Lösungsmittel wird im Sovirel-Reaktor abdestilliert (Badtemperatur <60 °C) solange der Rückstand gerührt werden kann. Anschliessend wird der ölige Rückstand mit 200 ml Methyl-tert-butylether (MTBE) aufgenommen, auf 0 °C abgekühlt und filtriert. Der Filterrückstand wird 2 x mit 100 ml MTBE und 2 x mit 50 ml Petrolether (50-70 °C) gewaschen. Der leicht grau gefärbte Feststoff wird mit 500 ml Wasser aufgenommen, mit verdünnter Salzsäure angesäuert und der Niederschlag filtriert und getrocknet.

Ausbeute: 58,1 g ≙ 86,3 % der Theorie.
Reinheit: >98 % nach $^{13}$C-Kernresonanzspektrum.
Schmelzpunkt: 72-75 °C.

Beispiel 13: 1-Phenyl-icosan-1,3-dion

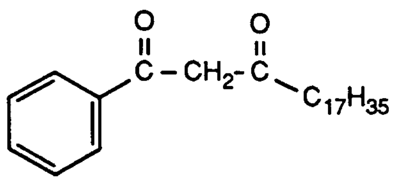

Analog zu Verfahrensweise, die im Beispiel 12 beschrieben ist, werden 99 g Stearinsäuremethylester und 36 g Acetophenon kondensiert.

Aufarbeitung Methode A:

Das Lösungsmittel wird am Rotationsverdampfer abdestilliert (Badtemperatur <60 °C) und der Rückstand mit 1000 ml Wasser aufgenommen. Anschliessend wird mit verdünnter Salzsäure angesäuert, der Niederschlag filtriert, mit 350 ml Methanol gewaschen und getrocknet (109,1 g = 94 % der Theorie). Das Rohprodukt wird aus 600 ml siedendem Methanol fraktioniert kristallisiert.

Ausbeute: 78,8 g ≙ 68 % der Theorie.
Reinheit: >98 % nach $^{13}$C-Kernresonanzspektrum.
Schmelzpunkt: 60-64 °C.

Aufarbeitung Methode B:

Das Lösungsmittel wird im Sovirel-Reaktor abdestilliert (Badtemperatur <60 °C), solange der Rückstand gerührt werden kann. Anschliessend wird der ölige Rückstand unter Rühren mit 200 ml Methanol versetzt, auf 20 °C abgekühlt und filtriert. Der Filterrückstand wird 2 x mit 100 ml Methanol gewaschen. Der leicht grau gefärbte Feststoff (Natriumsalz des Diketons) wird mit 750 ml Wasser aufgenommen, mit verdünnter Salzsäure angesäuert und der Niederschlag filtriert und getrocknet.

Ausbeute: 87,6 g ≙ 75,5 % der Theorie.
Schmelzpunkt: 60-64 °C.

Beispiel 14: 1-Phenyl-octadecan-1,3-dion

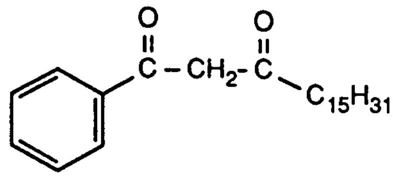

In einer Apparatur wie in Beispiel 1 werden 31,7 g Natrium-tert.-butylat, 150 g DMSO und 150 g Tetrahydrofuran (THF) vorgelegt und auf 0 °C abgekühlt. Anschliessend werden 89 g Palmitinsäuremethylester und 36 g Acetophenon als Gemisch über einen Zeitraum von 45 Minuten zugetropft. Das Reaktionsgemisch wird dann ca. 45-60 Minuten bei 30 °C gerührt, das Lösungsmittel am Rotationsverdampfer abdestilliert (Badtemperatur <60 °C) und der Rückstand mit 1000 ml Wasser aufgenommen. Anschliessend wird mit verdünnter Salzsäure angesäuert, der Niederschlag filtriert, mit Wasser gewaschen und getrocknet.

Das Rohprodukt (110,6 g) wird einmal aus 400 ml Methanol fraktioniert kristallisiert.

Ausbeute: 68,9 g ≙ 64,0 % der Theorie.
Reinheit: >98 % nach $^{13}C$-Kernresonanzspektrum.
Schmelzpunkt: 62-65 °C.

Beispiel 15: 1,4-Diphenyl-butan-1,3-dion

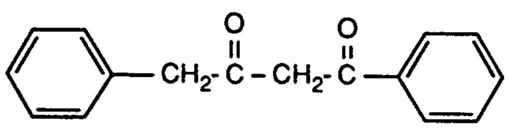

Verfahren wie in Beispiel 1 beschrieben. Anstatt Natriumhydrid werden 31,7 g Natrium-tert.-butylat und anstatt Benzoesäuremethylester werden 54,2 g Phenylessigsäureethylester eingesetzt.

Ausbeute: 60,7 g ≙ 84,9 % der Theorie, gelbe wachsartige Substanz, die aus 200 ml i-Propanol/30 ml Wasser umkristallisert wird. Schmelzpunkt 47-48 °C.

Beispiel 16: Temperaturverhalten des Verfahrens

Alle Reaktionen werden am Beispiel des Dibenzoylmethans (Produkt von Beispiel 1) in Dimethylsulfoxid/Dioxan mit Natriummethylat als Base + 3 % Methanol durchgeführt. Es wird nach dem Verfahren gemäss Beispiel 1 gearbeitet. Die Reaktionstemperatur wird von 0 bis 70 °C variiert.

Die Ergebnisse sind in Tabelle 1 wiedergegeben.

Tabelle 1

| Reaktionstemperatur in °C | Ausbeute in % | Reinheit in % |
|---|---|---|
| 0 | 89,2 | 99 |
| 10 | 91,4 | 97 |
| 20 | 90,3 | 98 |
| 30 | 90,3 | 94 |
| 50 | 80,8 | 99 |
| 70 | 82,4 | 96 |

Die Versuche zeigen, dass mit einer Temperatursteigerung über 50 °C keine Erhöhung der Ausbeute mehr zu erzielen ist.

**Patentansprüche**

**1.** Verfahren zur Herstellung von linearen 1,3-Diketonen der allgmeinen Formel I

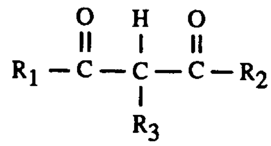

(I)

worin
$R_1$ und $R_2$ unabhängig voneinander für $C_1$-$C_{20}$-Alkyl, Phenyl, durch Halogen, Hydroxy, $NO_2$, $C_1$-$C_4$-Alkyl und/oder $C_1$-$C_4$-Alkoxy substituiertes Phenyl, $C_7$-$C_9$-Phenylalkyl oder für einen Rest der Formel II stehen

$-A-X-R_4$ (II)

wobei
A $C_1$-$C_{12}$-Alkylen, Phenylen, durch Halogen, Hydroxy, $NO_2$, $C_1$-$C_4$-Alkyl und/oder $C_1$-$C_4$-Alkoxy substituiertes Phenylen oder durch Hydroxy, Halogen oder/und Alkoxy substituiertes $C_1$-$C_{12}$-Alkylen bedeutet,
X für Sauerstoff oder Schwefel steht und
$R_4$ Wasserstoff, $C_1$-$C_{18}$-Alkyl, Phenyl, durch Halogen, Hydroxy, $C_1$-$C_4$-Alkyl, $NO_2$ und/oder $C_1$-$C_4$-Alkoxy substituiertes Phenyl oder $C_7$-$C_9$-Phenylalkyl bedeutet und
$R_3$ Wasserstoff, $C_1$-$C_{20}$-Alkyl, Phenyl, durch Halogen, Hydroxy, $C_1$-$C_4$-Alkyl, $NO_2$ und/oder $C_1$-$C_4$-Alkoxy substituiertes Phenyl oder $C_7$-$C_9$-Phenylalkyl darstellt, durch Claisen-Kondensation von Ketonen der Formel III

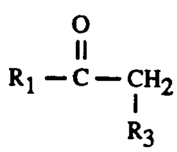

(III)

mit Estern der Formel IV

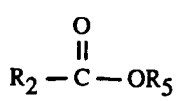

(IV)

worin $R_5$ für $C_1$-$C_5$-Alkyl, Phenyl, oder durch Halogen, $C_1$-$C_4$-Alkyl oder Hydroxy substituiertes Phenyl steht;
oder, wenn $R_2$ in Formel I $-(CH_2)_mOH$ bedeutet, auch mit cyclischen Estern der Formel V

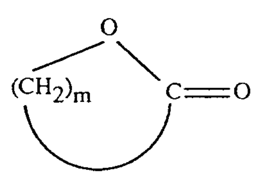

(V)

in der m 2 bis 10 bedeutet,

in Gegenwart eines Alkali- oder Erdalkalimetallhydrids oder $C_1$-$C_5$-Alkali- oder Erdalkalimetallakoholats als Base, dadurch gekennzeichnet, dass man die Umsetzung in einem Gemisch von Dimethylsulfoxid mit mindestens einem unter den Reaktionsbedinungen inerten, organischen Lösungmittel durchführt, wobei als inerte organische Lösungsmittel Dioxan, Tetrahydrofuran, Diethylenglycoldimethylether, Methyl-tert-butylether, Toluol oder N-Methylpyrrolidon eingesetzt werden.

**2.** Verfahren nach Anspruch 1, worin
$R_1$ und $R_2$ unabhängig voneinander $C_1$-$C_{20}$-Alkyl, Phenyl, ($C_1$-$C_4$-Alkyl)-phenyl oder einen Rest der Formel II bedeuten,
A für $C_1$-$C_6$-Alkylen steht,
$R_4$ Wasserstoff, $C_1$-$C_{18}$-Alkyl, Phenyl oder ($C_1$-$C_4$-Alkyl)-phenyl darstellt und
$R_3$ für Wasserstoff und $C_1$-$C_4$-Alkyl steht.

**3.** Verfahren nach Anspruch 2, worin
$R_1$ und $R_2$ unabhängig voneinander $C_1$-$C_{18}$-Alkyl, Phenyl oder einen Rest der Formel II bedeuten,
$R_4$ Wasserstoff, Phenyl oder $C_1$-$C_{18}$-Alkyl darstellt und
$R_3$ für Wasserstoff steht.

**4.** Verfahren nach Anspruch 1, worin der Anteil von Dimethylsulfoxid in der Mischung mit mindestens einem unter den Reaktionsbedingungen inerten, organischen Lösungsmittel 10 bis 80 % beträgt.

**5.** Verfahren nach Anspruch 1, worin die Claisen-Kondensationsreaktion bei Temperaturen zwischen -20 und +70 °C durchgeführt wird.

**6.** Verfahren nach Anspruch 5, worin die Reaktionstemperaturen zwischen -5 und +40 °C liegen.

**7.** Verfahren nach Anspruch 1, worin die Reaktionszeiten der Claisen-Kondensation zwischen 0,5 und 5 Stunden liegen.

**8.** Verfahren nach Anspruch 1, dadurch gekennzeichnet, dass das Alkali- oder Erdalkalimetallsalz des Diketons direkt aus der Reaktionslösung ausfällt, isoliert und durch Hydrolyse mit verdünnter Säure das reine Diketon erhalten wird.

**9.** Verfahren nach Anspruch 1, worin als Base ein Natriumalkoholat, insbesondere $NaOCH_3$ oder NaO-t-$C_4H_9$, oder NaH eingesetzt wird.

## Claims

**1.** A process for the preparation of a linear 1,3-diketone of general formula I

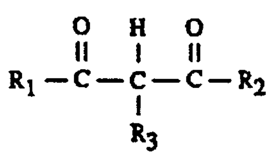

(I)

wherein
$R_1$ and $R_2$ are each independently of the other $C_1$-$C_{20}$alkyl, phenyl or phenyl which is substituted by halogen, hydroxyl, $NO_2$, $C_1$-$C_4$alkyl and/or $C_1$-$C_4$alkoxy, or are $C_7$-$C_9$phenylalkyl or a radical of formula II

-A-X-$R_4$ (II)

wherein
A is $C_1$-$C_{12}$alkylene, phenylene or phenylene which is substituted by halogen, hydroxyl, $NO_2$, $C_1$-$C_4$alkyl and/or $C_1$-$C_4$alkoxy, or is $C_1$-$C_{12}$alkylene which is substituted by hydroxyl, halogen and/or alkoxy,

X is oxygen or sulfur, and
$R_4$ is hydrogen, $C_1$-$C_{18}$alkyl, phenyl or phenyl which is substituted by halogen, hydroxyl, $C_1$-$C_4$alkyl, $NO_2$ and/or $C_1$-$C_4$alkoxy, or is $C_7$-$C_9$phenylalkyl, and
$R_3$ is hydrogen, $C_1$-$C_{20}$alkyl, phenyl or phenyl which is substituted by halogen, hydroxyl, $C_1$-$C_4$alkyl, $NO_2$ and/or $C_1$-$C_4$alkoxy, or is $C_7$-$C_9$phenylalkyl,
by Claisen condensation of a ketone of formula III

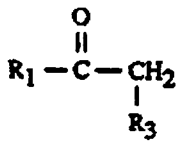

(III)

with an ester of formula IV

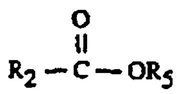

(IV)

wherein $R_5$ is $C_1$-$C_5$alkyl, phenyl or phenyl which is substituted by halogen, $C_1$-$C_4$alkyl or hydroxyl;
or else, if $R_2$ in formula I is $-(CH_2)_mOH$, with a cyclic ester of formula V

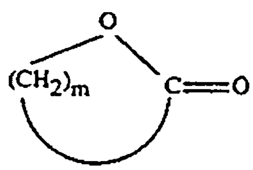

(V)

in which m is 2 to 10,
in the presence of a hydride of an alkali metal or alkaline earth metal or of a $C_1$-$C_5$alcoholate of an alkali metal or alkaline earth metal as base, which process comprises carrying out the reaction in a mixture of dimethyl sulfoxide and at leat one organic solvent which is inert under the reaction conditions, the inert organic solvent employed being dioxane, tetrahydrofuran, diethylene glycol dimethyl ether, methyl tert-butyl ether, toluene or N-methylpyrrolidone.

**2.** A process according to claim 1, wherein $R_1$ and $R_2$ are each independently of the other $C_1$-$C_{20}$alkyl, phenyl, ($C_1$-$C_4$alkyl)phenyl or a radical of formula II,
A is $C_1$-$C_6$alkylene,
$R_4$ is hydrogen, $C_1$-$C_{18}$alkyl, phenyl or ($C_1$-$C_4$alkyl)phenyl, and
$R_3$ is hydrogen or $C_1$-$C_4$alkyl.

**3.** A process according to claim 2, wherein $R_1$ and $R_2$ are each independently of the other $C_1$-$C_{18}$alkyl, phenyl or a radical of formula II,
$R_4$ is hydrogen, phenyl or $C_1$-$C_{18}$alkyl, and
$R_3$ is hydrogen.

**4.** A process according to claim 1, wherein the amount of dimethyl sulfoxide in the mixture with at least one organic solvent which is inert under the reaction conditions is 10 to 80 %.

**5.** A process according to claim 1, wherein the Claisen condensation reaction is carried out at temperatures of between -20 and +70 °C.

**6.** A process according to claim 5, wherein the reaction temperatures are between -5 and +40 °C.

7. A process according to claim 1, wherein the reaction times of the Claisen condensation are between 0.5 and 5 hours.

8. A process according to claim 1, wherein the alkali metal salt or alkaline earth metal salt of the diketone is precipitated direct from the reaction solution and isolated, and the pure diketone is obtained by hydrolysis with dilute acid.

9. A process according to claim 1, wherein the base employed is a sodium alcoholate, especially $NaOCH_3$ or $NaO\text{-}t\text{-}C_4H_9$, or NaH.

## Revendications

1. Procédé pour préparer des 1,3-dicétones linéaires de formule générale I

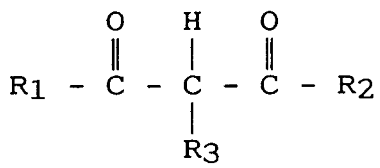 (I)

dans laquelle
$R_1$ et $R_2$, indépendamment l'un de l'autre, sont chacun un radical alkyle en $C_1$-$C_{20}$, phényle, phényle substitué par des substituants halogéno, hydroxy, $NO_2$, alkyle en $C_1$-$C_4$ et/ou alcoxy en $C_1$-$C_4$, phénylalkyle en $C_7$-$C_9$ ou encore un radical de formule II

$-A-X-R_4$ (II)

dans laquelle
A est un radical alkylène en $C_1$-$C_{12}$, phénylène, phénylène substitué par des substituants halogéno, hydroxy, $NO_2$, alkyle en $C_1$-$C_4$ et/ou alcoxy en $C_1$-$C_4$, ou encore alkylène en $C_1$-$C_{12}$ substitué par des substituants hydroxy, halogéno et/ou alcoxy,
X est un oxygène ou un soufre, et
$R_4$ est un hydrogène, un radical alkyle en $C_1$-$C_{18}$, phényle, phényle substitué par des substituants halogéno, hydroxy, alkyle en $C_1$-$C_4$, $NO_2$ et/ou alcoxy en $C_1$-$C_4$, ou encore un radical phénylalkyle en $C_7$-$C_9$, et
$R_3$ est un hydrogène, un radical alkyle en $C_1$-$C_{20}$, phényle, phényle substitué par des substituants halogéno, hydroxy, alkyle en $C_1$-$C_4$, $NO_2$ et/ou alcoxy en $C_1$-$C_4$, ou encore un radical phénylalkyle en $C_7$-$C_9$,
par condensation de Claisen de cétones de formule III

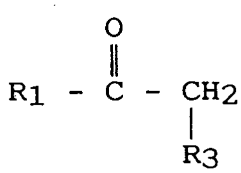 (III)

avec des esters de formule IV

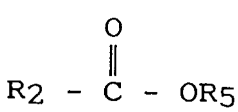 (IV)

dans laquelle $R_5$ est un radical alkyle en $C_1$-$C_5$, phényle ou phényle substitué par des substituants halogéno, alkyle en $C_1$-$C_4$ ou hydroxy ;

ou bien, quand $R_2$, dans la formule I est $-(CH_2)_mOH$, avec des esters cycliques de formule V

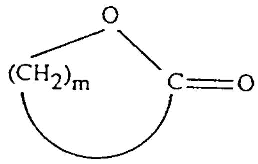

(V)

dans laquelle m vaut de 2 à 10,

en présence d'un hydrure d'un métal alcalin ou alcalino-terreux ou d'un alcoolate en $C_1$-$C_5$ d'un métal alcalin ou alcalino-terreux, servant de base, caractérisé en ce qu'on met en oeuvre la réaction dans un mélange de diméthylsulfoxyde et d'au moins un solvant organique inerte dans les conditions de la réaction, auquel cas on utilise comme solvant organique inerte le dioxanne, le tétrahydrofuranne, l'éther diméthylique du diéthylèneglycol, l'oxyde de méthyle et de tert-butyle, le toluène ou encore la N-méthylpyrrolidone.

**2.** Procédé selon la revendication 1, dans lequel

$R_1$ et $R_2$, indépendamment l'un de l'autre, sont des radicaux alkyle en $C_1$-$C_{20}$, phényle, (alkyle en $C_1$-$C_4$)-phényle ou un radical de formule II,

A est un radical alkylène en $C_1$-$C_6$,

$R_4$ est un hydrogène ou un radical alkyle en $C_1$-$C_{18}$, phényle ou (alkyle en $C_1$-$C_4$)-phényle, et

$R_3$ est un hydrogène et un radical alkyle en $C_1$-$C_4$.

**3.** Procédé selon la revendication 2, dans lequel

$R_1$ et $R_2$, indépendamment l'un de l'autre, sont des radicaux alkyle en $C_1$-$C_{18}$, phényle, ou un radical de formule II,

$R_4$ est un hydrogène ou un radical phényle ou alkyle en $C_1$-$C_{18}$, et

$R_3$ est un hydrogène.

**4.** Procédé selon la revendication 1, dans lequel la quantité de diméthylsulfoxyde dans le mélange avec au moins un solvant organique inerte dans les conditions de la réaction est de 10 à 80 %.

**5.** Procédé selon la revendication 1, dans lequel la réaction de condensation de Claisen est mise en oeuvre à des températures comprises entre -20 et +70 °C.

**6.** Procédé selon la revendication 5, dans lequel les températures de réaction sont comprises entre -5 et +40 °C.

**7.** Procédé selon la revendication 1, dans lequel les temps de réaction de la condensation de Claisen sont compris entre 0,5 et 5 heures.

**8.** Procédé selon la revendication 1, caractérisé en ce que le sel d'un métal alcalin ou alcalino-terreux de la dicétone précipite directement de la solution réactionnelle, est isolé, puis on obtient la dicétone pure par hydrolyse avec un acide dilué.

**9.** Procédé selon la revendication 1, dans lequel on utilise comme base un alcoolate de sodium, notamment $NaOCH_3$ ou NaO-tert-$C_4H_9$, ou NaH.